# EUROPÄISCHE PATENTSCHRIFT

(11) **EP 1 107 703 B1**
(45) Veröffentlichungstag und Bekanntmachung des Hinweises auf die Patenterteilung: **02.07.2003**
(21) Anmeldenummer: 00964023.6
(22) Anmeldetag: 18.08.2000
(51) Int. Cl.: A61B 18/14, A61B 17/28

(54) **MEDIZINISCHES BIPOLARES INSTRUMENT ZUM SCHNEIDEN VON GEWEBE**
MEDICAL BIPOLAR INSTRUMENT FOR CUTTING TISSUE
INSTRUMENT MEDICAL BIPOLAIRE SERVANT A COUPER DES TISSUS

(30) Priorität: 28.09.1999 DE 19946527; 14.02.2000 DE 20002645 U
(43) Veröffentlichungstag der Anmeldung: 20.06.2001
(60) Teilanmeldung: 03009909.7
(73) Patentinhaber: Karl Storz GmbH & Co. KG, 78532 Tuttlingen (DE)
(72) Erfinder: BLOCHER, Martin, D-78532 Tuttlingen (DE); REMORGIDA, Valentino, I-16145 Genua (IT)
(74) Vertreter: Heuckeroth, Volker, Dr.
(86) Internationale Anmeldenummer: EP0008061
(87) Internationale Veröffentlichungsnummer: WO01022896

(56) Entgegenhaltungen:
- WO-A-99/05976
- WO-A-99/23960
- US-A- 5 637 111

## Beschreibung

Ein medizinisches bipolares Instrument zum Schneiden von Gewebe ist aus dem DE-Firmenkatalog der Firma Karl Storz GmbH & Co. KG, Tuttlingen, "STORZ Karl Storz-Endoskope", Band "Laparoskopie", 3. Ausgabe 1/1999, Seite BI-COA 10/2, bekannt.

Ein solches medizinisches bipolares Instrument dient bspw. in der laparoskopischen Chirurgie zum Präparieren von Gewebe im menschlichen oder tierischen Körper.

Beim Präparieren von Gewebe werden in das Gewebe Schnitte eingebracht. Da beim Einbringen von Schnitten Blutungen auftreten können, müssen diese gestillt werden.

Bei dem bekannten Instrument ist die Anwendung von bipolarem Hochfrequenzstrom auf die Blutstillung, d.h. Koagulation beschränkt. Das Einbringen von Schnitten in das Gewebe erfolgt dagegen auf mechanischem Wege, und zwar mittels einer skalpellartigen Klinge.

Das zuvor genannte bekannte Instrument weist ein erstes Arbeitselement in Form eines Mauls und ein zweites Arbeitselement ebenfalls in Form eines Mauls auf, die Seite an Seite voneinander beabstandet angeordnet sind, mit anderen Worten in der Art einer Zwillingszange. Das das erste Arbeitselement bildende Maul ist als mit Hochfrequenzstrom beaufschlagbare Elektrode und das das zweite Arbeitselement bildende zweite Maul als Gegenelektrode ausgebildet, wobei das erste Maul über eine entsprechende Stromzuführung mit dem einen Pol einer Hochfrequenzspannungsquelle und das zweite Maul über eine entsprechende Stormzuführung mit dem anderen Pol der Hochfrequenzspannungsquelle verbindbar ist. Das erste Maul und das zweite Maul sind durch ihre Beabstandung voneinander gegeneinander isoliert.

Ein damit vergleichbares Instrument ist aus der WO 97/17033 bekannt.

Beim Einschalten der Hochfrequenzspannungsquelle bildet sich ein Stromfluß durch das zwischen dem ersten Maul und dem zweiten Maul befindliche Gewebe aus, so daß der Koagulationseffekt auf den Bereich zwischen den beiden Mäulern beschränkt ist. Zum Koagulieren eines Gefäßes wird dieses sich quer zu den Mäulern erstreckend gefaßt.

Die Schneidfunktion dieses bekannten Instruments zum Schneiden von Gewebe, insbesondere zum Durchtrennen von Gefäßen, die in dem Gewebe vorhanden sein können, ist bei dem bekannten Instrument durch eine skalpellartige Klinge realisiert, die zwischen dem ersten Maul und dem zweiten Maul angeordnet ist, und die über eine separate Handhabe am proximalen Ende des Instruments in Längsrichtung nach vorn geschoben werden kann, um bspw. ein Gefäß, das sich quer zu den Mäulern erstreckend gefaßt wurde, zu durchtrennen. Die Schneidwirkung der Klinge ist rein mechanisch. Durch das Vorsehen der Klinge zwischen dem ersten Maul und dem zweiten Maul wird gegenüber anderen herkömmlichen bipolaren Instrumenten, mit denen ausschließlich koaguliert werden kann, das Einführen eines zusätzlichen Schneidelements, wie eine Schere oder ein Skalpell, in das Operationsgebiet und damit ein Instrumentenwechsel vermieden.

Dennoch erweist sich die Ausgestaltung dieses bekannten Instruments mit einer skalpellartigen Klinge zum Schneiden von Gewebe als nachteilig, wenn die Abmessungen des Instruments verkleinert werden sollen, d.h. dieses möglichst schmalbauend ausgebildet werden soll. Während das bekannte Instrument einen Instrumentendurchmesser von etwa 10 mm aufweist, sind bei bestimmten Anwendungen derartiger Instrumente Durchmesser von etwa 5 mm erstrebenswert.

Eine Reduzierung des Durchmessers des bekannten Instruments von 10 mm auf 5 mm ist jedoch aus folgenden Gründen nicht möglich. Da zwischen dem ersten Maul und dem zweiten Maul die skalpellartige Klinge Platz finden muß, weisen das erste Maul und das zweite Maul des bekannten Instruments bereits einen geringen Durchmesser von nur 3 mm auf und sind etwa 3 mm voneinander beabstandet. Eine Reduzierung des Gesamtdurchmessers der Anordnung aus erstem Maul, zweitem Maul und skalpellartiger Klinge auf 5 mm würde es erfordern, das erste Maul und das zweite Maul im Durchmesser auf weniger als 1 mm zu reduzieren. Mit derartigen Abmessungen ist jedoch die Stabilität der die Arbeitselemente bildenden Mäuler nicht mehr gewährleistet. Um mit dem Instrument Gewebe sicher präparieren zu können, ist jedoch eine ausreichende Stabilität des ersten und zweiten Arbeitselements erforderlich.

Aus dem eingangs genannten DE-Firmenkatalog ist auf Seite BI-COA 5/12 ein weiteres Instrument bekannt, das ein Faß- und Koagulationsinstrument, jedoch ohne Schneidfunktion, ist, dessen Arbeitselement ein oberes und ein unteres Maulteil aufweist. Die beiden Maulteile sind als Faßwerkzeuge ausgebildet und außerdem als Elektrode und Gegenelektrode, so daß sich zwischen den Maulteilen durch zwischen diesen gefaßtes Gewebe ein Stromfluß zum Koagulieren des Gewebes ausbilden kann.

Das Dokument US-A-5 637 111 offenbart ein Instrument gemäß oberbegriff des Anspruchs 1.

Aus der WO-A-99/23960 ist ferner ein Instrument zum Verschweißen von Blutgefäßen bekannt. In diesem Dokument wird zum Stand der Technik eine Koagulationszange beschrieben, die nur ein Maul mit zwei beweglichen Maulteilen aufweist, die auf ihrer einander zugewandten Seite jeweils eine Zahnung aufweisen, um ein zu schweißendes Blutgefäß sicher fassen zu können. Das Instrument kann auch im bipolaren Modus betrieben werden, wobei dann das eine Maulteil und das andere Maulteil als Gegenelektrode wirken. Mit der vorgesehenen Zahnung der beiden Maulteile kann jedoch nicht Gewebe geschnitten werden. In dem selben Dokument ist ein weiteres Instrument zum Verschweißen von Blutgefäßen offenbart, bei dem zur Erzielung einer Schneidfunktion eine hin- und herbewegbare Klinge zum Durchschneiden des verschweißten Blutgefäßes vorgesehen sein kann, mit der demnach mechanisch geschnitten wird. Alternativ dazu kann auch eine separate scherenartige Klinge vorgesehen sein, die in einem Schlitz in einem Maulteilarm drehbar ist. Auch bei diesem bekannten Instrument wird eine Schneidfunktion demnach mechanisch bewirkt.

Der Erfindung liegt allgemein die Aufgabe zugrunde, ein bipolares Instrument zum Schneiden von Gewebe der eingangs genannten Art zu schaffen, bei dem die Schneidfunktion auf andere Weise als auf mechanischem Wege realisiert ist.

Erfindungsgemäß wird diese Aufgabe durch ein Instrument gemäß Anspruch 1 gelöst.

Bei dem erfindungsgemäßen Instrument besteht die vorteilhafte Wirkung der Konzentration der Stromdichte darin, daß nunmehr auf elektrischem Wege ein Schnitt in das Gewebe eingebracht oder ein Gefäß in dem Gewebe durchtrennt werden kann. Gegenüber dem bekannten Instrument ist die Funktion des Schneidens demnach nicht durch ein mechanisch wirkendes Schneidinstrument, wie eine skalpellartige Klinge, sondern als "elektrische Schneide" realisiert. Das Einbringen eines Schnitts mittels des erfindungsgemäßen Instruments in Gewebe erfolgt dadurch, daß das Instrument von Hand so geführt bzw. bewegt wird, daß die sich zwischen dem zumindest einen Vorsprung und dem anderen Arbeitselement ausbildende "elektrische Schneide" durch das Gewebe bzw. durch das Gefäß bewegt wird. Der Schneidvorgang selbst erfolgt dabei im wesentlichen berührungslos.

Die Anwendung der Erfindung bei dem bekannten Instrument hat den besonderen Vorteil, daß das das erste Arbeitselement bildende

Maul und das das zweite Arbeitselement bildende Maul wegen des Wegfalls des mechanischen Schneidinstruments zwischen beiden im Vergleich zu dem bekannten Instrument durchmessergrößer und damit stabiler ausgestaltet werden können, selbst wenn der Gesamtdurchmesser des Instruments auf wenige, bspw. 5 mm reduziert wird. In diesem Fall ergibt sich damit einhergehend als noch weiterer Vorteil der erfindungsgemäßen Ausgestaltung, daß zum Schneiden von Gewebe keine weitere Handhabe am Instrument vorgesehen werden muß, wie dies bei dem bekannten Instrument der Fall ist, um die skalpellartige Klinge vor- und zurückzubewegen. Bei dem erfindungsgemäßen Instrument erfolgt das Schneiden dagegen mit den Arbeitselementen selbst, so daß auch der konstruktive Aufwand des erfindungsgemäßen Instruments vorteilhaft reduziert ist. Mit dem Instrument kann zunächst zwischen den Maulteilen Gewebe gefaßt und koaguliert werden. Zum anschließenden Einbringen eines Schnittes oder zum Durchtrennen eines Gefäßes werden die Maulteile geöffnet, das Instrument ein Stück zurückgezogen, die Maulteile geschlossen und das Instrument dann mit den Maulteilen so geführt, daß diese sich zwischen dem zumindest einen Vorsprung des einen Mauls und des anderen Mauls ausbildende "elektrische Schneide" durch das Gewebe dringt, um dieses zu schneiden.

In einer bevorzugten Ausgestaltung weisen sowohl das erste Arbeitselement als auch das zweite Arbeitselement jeweils zumindest einen Vorsprung auf.

Diese Maßnahme führt vorteilhafterweise zu einer weiteren Erhöhung der Konzentration der Stromdichte zwischen dem ersten Arbeitselement und dem zweiten Arbeitselement, wodurch die elektrische Schneidwirkung noch weiter verbessert werden kann.

Dabei ist es bevorzugt, wenn der zumindest eine Vorsprung des ersten Arbeitselements dem zumindest einen Vorsprung des zweiten Arbeitselements gegenübersteht.

Hierbei ist von Vorteil, daß die Schnittlinie, die durch die sich gegenüberstehenden Vorsprünge definiert wird, sehr exakt definiert ist. Die Vorsprünge können sich dabei in Längsrichtung der Arbeitselemente oder in einer Schräg- oder Querrichtung gegenüberstehen. Bspw. kann bei sich in Querrichtung der Arbeitselemente gegenüberstehenden Vorsprüngen durch axiales Verschieben des Instruments oder durch eine Bewegung quer zur Längsrichtung der Arbeitselemente geschnitten werden, und bei sich in Längsrichtung gegenüberstehenden Vorsprüngen kann durch eine Drehung des Instruments um seine Längsachse geschnitten werden.

Bei sich in Arbeitselementlängsrichtung gegenüberstehenden Vorsprüngen ist es weiterhin bevorzugt, wenn das erste Arbeitselement das zweite Arbeitselement in Arbeitselementlängsrichtung überragt.

In einer weiteren bevorzugten Ausgestaltung ist das freie Ende des zumindest einen Vorsprungs als Spitze ausgebildet.

Mit einer Ausbildung des freien Endes des zumindest einen Vorsprungs als Spitze können wegen des auftretenden Spitzeneffektes besonders hohe Stromdichten und damit eine besonders gute Schneidwirkung erzielt werden. Des weiteren kann die durch den zumindest einen Vorsprung definierte "elektrische Schneide" besonders präzise definiert werden.

Das freie Ende des zumindest einen Vorsprungs kann jedoch auch als Kante quer zur Arbeitselementrichtung ausgebildet sein, um die gesamte zwischen dem ersten Arbeitselement und dem zweiten Arbeitselement befindliche Dicke des Gewebes besser schneiden zu können. Dies kann auch dadurch erreicht werden, daß an beiden Arbeitselementen auf axial etwa gleicher Höhe jeweils ein Vorsprung angeordnet ist, deren freies Ende jeweils wiederum als Spitze ausgebildet ist, wie vorstehend beschrieben.

In einer weiteren bevorzugten Ausgestaltung kann der zumindest eine Vorsprung jedoch auch abgerundet sein.

Bei dieser Ausgestaltung wird zwar keine zu hohe Stromdichte wie bei einer Spitze erzielt, jedoch kann durch diese Ausgestaltung vorteilhafterweise die elektrische Schneidwirkung auf einen Streifen entsprechend der Breite des abgerundeten freien Endes verbreitert werden.

Vorteilhafterweise kann ein besonders scharfer und wohldefinierter kurzer Schnitt gesetzt werden, wenn das freie Ende des zumindest einen Vorsprungs des ersten Arbeitselements und das freie Ende des zumindest einen Vorsprungs des zweiten Arbeitselements einen minimalen Abstand voneinander aufweisen, da der Stromfluß dann optimal auf die kurze Verbindungsstrecke zwischen den Vorsprüngen mit hoher Dichte konzentriert ist. "Minimaler" Abstand bedeutet, daß der Abstand der Arbeitselemente im Bereich der Vorsprünge kleiner ist als im übrigen Bereich der Arbeitselemente.

Ein solcher minimaler Abstand kann bspw. etwa 1 mm oder darunter betragen.

Auch kann das freie Ende des zumindest einen Vorsprungs bevorzugt als längliche Kante ausgebildet sein.

Dabei ist es weiterhin bevorzugt, wenn der zumindest eine Vorsprung keilartig schräg zur Arbeitselementlängsrichtung verläuft. Ebenso bevorzugt ist es dabei, wenn sowohl an dem ersten Arbeitselement als auch an dem zweiten Arbeitselement zumindest ein als längliche Kante ausgebildeter Vorsprung vorgesehen ist, wobei die Kanten jeweils schräg zur Arbeitselementlängsrichtung verlaufend ausgebildet sind, wobei die sich gegenüberliegenden Kanten gegensinnig zueinander schräg verlaufen.

Aus dem Vorstehenden geht hervor, daß durch eine entsprechende Wahl der Anordnung des zumindest einen Vorsprungs, bevorzugt mehrerer Vorsprünge, ein elektrisches Schneiden von Gewebe entlang definierter Schnittlinien ermöglicht wird. Im Rahmen der Erfindung ist es daher möglich, durch eine entsprechende Wahl der Anzahl und Positionierung eines oder mehrerer Vorsprünge eine oder mehrere definierte Schnittlinien zum elektrischen Schneiden von Gewebe zu erzeugen.

Eine besonders vorteilhafte Anpassung des erfindungsgemäßen Instruments an den jeweiligen Anwendungszweck wird in diesem Sinne dadurch erreicht, daß das erste Arbeitselement und das zweite Arbeitselement auswechselbar sind. Es können demnach verschiedene Arbeitselemente mit einer entsprechenden Anzahl und Verteilung von Vorsprüngen bereitgehalten werden.

In einer weiteren bevorzugten Ausgestaltung ist der zumindest eine Vorsprung an dem ersten Maulteil des einen Mauls angeordnet, und ist zumindest ein zweiter Vorsprung an dem zweiten Maulteil desselben Mauls angeordnet.

Hierbei ist von Vorteil, daß sich bei geschlossenen Maulteilen oder verstärkt bei geringfügig geöffneten Maulteilen eine "elektrische Schneide" mit einer flächigen und nicht nur linienförmigen Ausdehnung zwischen den Arbeitselementen des Instruments ausbildet. Besonders vorteilhaft ist es, wenn das andere Maul ebenso eine Anordnung von zumindest zwei Vorsprüngen aufweist, von denen ein Vorsprung an dem ersten Maulteil und ein zweiter Vorsprung an dem zweiten Maulteil des anderen Mauls angeordnet sind.

In einer weiteren bevorzugten Ausgestaltung ist der zumindest eine Vorsprung des ersten Mauls an einem ersten Maulteil des ersten Mauls und der zumindest eine Vorsprung des zweiten Mauls an demjenigen Maulteil des zweiten Mauls angeordnet, das dem ersten Maulteil des ersten Mauls diametral gegenüberliegt.

Bei dieser Ausgestaltung wird eine Schnittlinie erzielt, deren Richtung schräg zur von dem ersten und zweiten Maul aufgespannten Hauptebene (Horizontalebene) verläuft.

Bei beiden vorgenannten Ausgestaltungen ist es bevorzugt, wenn die zumindest beiden Vorsprünge in Arbeitselementlängsrichtung auf der gleichen Höhe angeordnet sind.

Der Vorteil dieser Anordnung besteht darin, daß an jeweils einer gleichen Gewebestelle von beiden Seiten her ein präziser Schnitt durchgeführt werden kann.

Weitere Vorteile ergeben sich aus der nachfolgenden Beschreibung und der beigefügten Zeichnung.

Ausführungsbeispiele der Erfindung sind in der Zeichnung dargestellt und werden in der nachfolgenden Beschreibung näher erläutert. Es zeigen:
- Fig. 1: ein medizinisches bipolares Instrument zum Schneiden von Gewebe in einer Gesamtdarstellung in Seitenansicht in zwei Teilbildern, wobei ein distaler Abschnitt des Instruments im vergrößerten Maßstab dargestellt ist;
- Fig. 2: eine perspektivische Darstellung des distalen Abschnitts des Instruments in Fig. 1 in einem noch weiter vergrößerten Maßstab;
- Fig. 3: ein weiteres Ausführungsbeispiel für ein erstes und zweites Arbeitselement des Instruments in Fig. 1 in einer perspektivischen Darstellung in stark vergrößertem Maßstab;
- Fig. 4: ein noch weiteres Ausführungsbeispiel für ein erstes und zweites Arbeitselement für das Instrument in Fig. 1 in perspektivischer Darstellung in stark vergrößertem Maßstab;
- Fig. 5a) und b): eine schematische Draufsicht (Fig. 5a)) und Vorderansicht (Fig. 5b)) des ersten und zweiten Arbeitselements aus Fig. 2;
- Fig. 6a) und b): = eine schematische Draufsicht (Fig. 6a)) und Vorderansicht (Fig. 6b)) des ersten und zweiten Arbeitselements aus Fig. 4;
- Fig. 7a) und b): eine schematische Draufsicht (Fig. 7a)) und Seitenansicht (Fig. 7b)) eines noch weiteren Ausführungsbeispiels eines ersten und zweiten Arbeitselements zur Verwendung in dem Instrument gemäß Fig. 1;
- Fig. 8: ein gegenüber Fig. 7 abgewandeltes Ausführungsbeispiel für ein erstes und zweites Arbeitselements in einer Draufsicht;
- Fig. 9: ein weiteres Ausführungsbeispiel eines bipolaren Instruments zum Schneiden von Gewebe im Bereich seines distalen Endes in Seitenansicht;
- Fig. 10: das Instrument in Fig. 9, wobei das eine Arbeitselement weggelassen wurde;
- Fig. 11: eine Draufsicht auf das distale Ende des Instruments in Fig. 9 und 10;

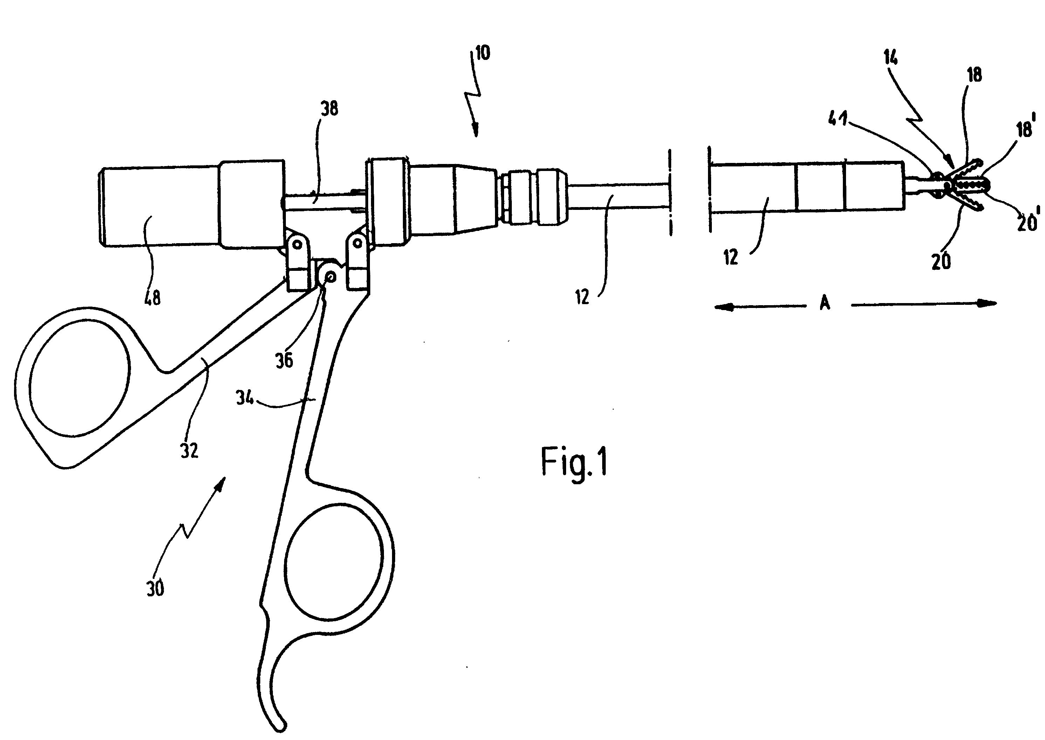

In Fig. 1 ist ein mit dem allgemeinen Bezugszeichen 10 versehenes medizinisches bipolares Instrument zum Schneiden von Gewebe dargestellt. Mit dem Instrument 10 kann, wie nachfolgend näher beschrieben wird, Gewebe nicht nur geschnitten, sondern auch gefaßt und koaguliert werden. Das Instrument 10 wird im Rahmen der minimal-invasiven Chirurgie zum Präparieren von Gewebe verwendet.

Das Instrument 10 weist einen langerstreckten Schaft 12 auf, dessen in Fig. 1 mit A bezeichneter distaler Abschnitt in vergrößertem Maßstab dargestellt ist.

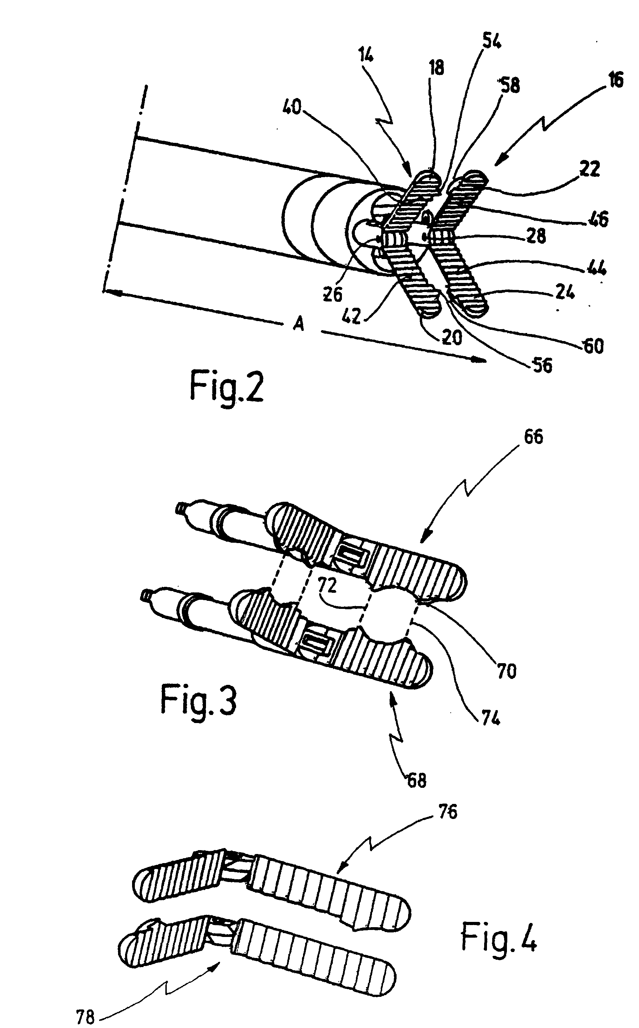

Der Abschnitt A ist in Fig. 2 perspektivisch und noch weiter vergrößert dargestellt.

Der Schaft 12 ist als Rohrschaft ausgebildet. Am distalen Ende des Schaftes 12 sind ein erstes Arbeitselement 14 und ein dazu benachbartes zweites Arbeitselement 16 angeordnet, wobei in der Seitenansicht in Fig. 1 nur das rechte erste Arbeitselement 14 zu sehen ist. Die Arbeitselemente 14 und 16 sind jeweils als Maul ausgebildet.

Das erste Arbeitselement 14 weist ein erstes Maulteil 18 und ein zweites Maulteil 20 auf.

Das zweite Arbeitselement 16 weist entsprechend ein erstes Maulteil 22 und ein zweites Maulteil 24 auf.

Das erste Maulteil 18 und das zweite Maulteil 20 sind relativ zueinander beweglich. In dem in Fig. 2 gezeigten Ausführungsbeispiel sind dabei sowohl das erste Maulteil 18 als auch das zweite Maulteil 20 beweglich, und zwar sind beide um eine Schwenkachse 26 verschwenkbar. Ebenso sind das erste Maulteil 22 und das zweite Maulteil 24 des zweiten Arbeitselements 16 relativ zueinander beweglich, und zwar sind beide Maulteile 22 und 24 um eine Schwenkachse 28 verschwenkbar.

In Fig. 1 ist das Arbeitselement 14 mit seinen Maulteilen 18 und 20 in Offenlage und mit 18' und 20' in seiner Schließlage dargestellt.

Wie aus Fig. 2 hervorgeht, sind das erste Arbeitselement 14 und das zweite Arbeitselement 16 benachbart, und zwar Seite an Seite voneinander beabstandet angeordnet. Speziell sind das erste Arbeitselement 14 und das zweite Arbeitselement 16 parallel zueinander ausgerichtet und lassen sich synchron öffnen und schließen.

Zum Öffnen und Schließen des ersten Arbeitselements 14 und des zweiten Arbeitselements 16 weist das Instrument 10 eine Handhabe 30 auf, die zwei relativ zueinander bewegliche Griffteile 32 und 34 aufweist. Das Griffteil 32 und das Griffteil 34 sind dazu über eine Schwenkachse 36 gelenkig miteinander verbunden.

Das Griffteil 32 ist über einen Kraftübertragungsmechanismus, der zwei Zug- und Schubstangen umfaßt, von denen in Fig. 1 nur die rechte Zug- und Schubstange 38 zu sehen ist, mit dem ersten Arbeitselement 14 bzw. dem zweiten Arbeitselement 16 kraftschlüssig verbunden. Die Zug- und Schubstange 38 -sowie die parallel dazu verlaufende, in Fig. 1 nicht sichtbare Schub- und Zugstange sind jeweils über eine Kniehebelanordnung 41 mit dem entsprechenden Arbeitselement 14 bzw. 16 verbunden.

Durch Zusammendrücken des ersten Griffteils 32 und des zweiten Griffteils 34 werden das das erste Arbeitselement 14 bildende Maul und das das zweite Arbeitselement 16 bildende Maul synchron geschlossen, und durch Auseinanderbewegen der Griffteile 32 und 34 werden das erste Maul und das zweite Maul synchron geöffnet.

Gemäß einer ersten Funktion des ersten Arbeitselements 14 und des zweiten Arbeitselements 16 sind die Maulteile 18 und 20 bzw. 22 und 24 als Faßwerkzeuge ausgebildet, d.h. Innnenseiten 40, 42, 44 und 46 der entsprechenden Maulteile 18, 20, 22 und 24 sind flächig ausgebildet und zusätzlich mit Zahnungen versehen, um die Griffigkeit der Innenseiten zum Fassen und sicheren Festhalten von Gewebe zu verbessern.

Gemäß einer weiteren Funktion des ersten Arbeitselements 14 und des zweiten Arbeitselements 16 ist das erste Arbeitselement 14 als mit Hochfrequenzstrom beaufschlagbare Elektrode und das zweite Arbeitselement 16 als Gegenelektrode ausgebildet, um zwischen den Maulteilen 18 und 20 bzw. 22 und 24 gefaßtes Gewebe zu koagulieren. Die Maulteile 18 und 20 bzw. 22 und 24 sind dazu zumindest an ihren Innenseiten 40 bis 46 oder insgesamt metallisch und damit stromführend ausgebildet.

Das erste Arbeitselement 14 ist über eine Stromzuführung, die durch die zugehörige Zug- und Schubstange gebildet ist, mit einem ersten nicht näher dargestellten Kontaktpol eines Stekkeranschlusses 48 am proximalen Ende des Instruments 10 in Fig. 1 verbunden, während das zweite Arbeitselement 16 über die andere Zug- und Schubstange als Stromzuführung mit dem anderen Kontaktpol des Steckeranschlusses 48 verbunden ist. An den Steckeranschluß 48 ist ein Hochfrequenzstromkabel (nicht dargestellt) anschließbar, das mit einer externen nicht dargestellten Hochfrequenzspannungsquelle verbindbar oder verbunden ist.

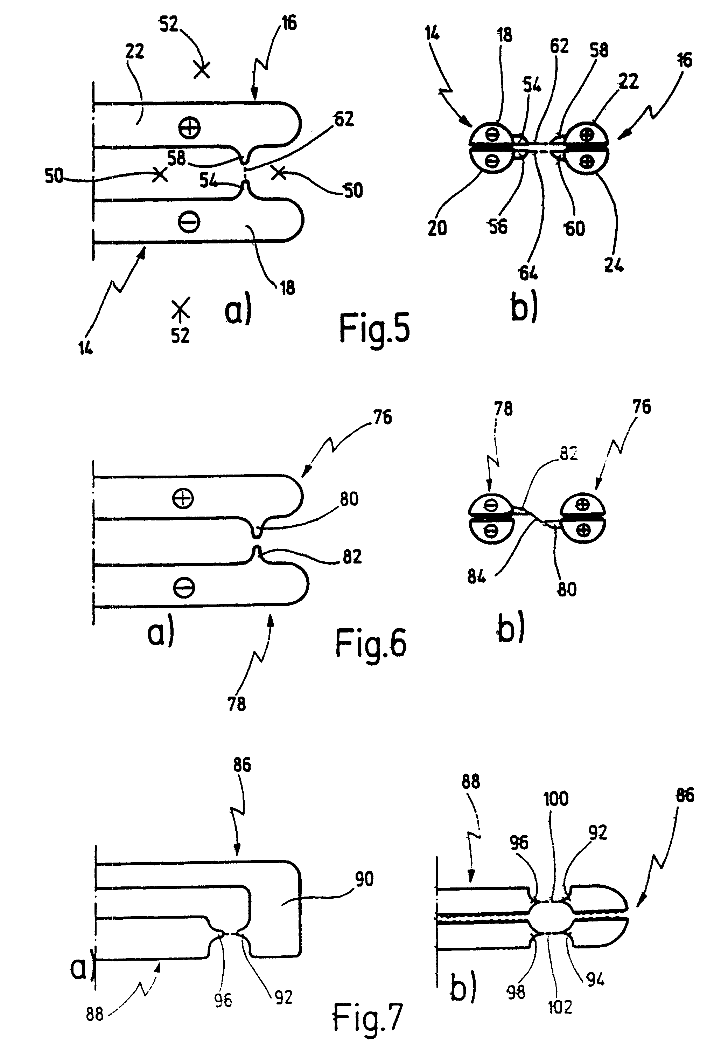

In Fig. 5, die das erste Arbeitselement 14 und das zweite Arbeitselement 16 in Alleinstellung schematisch darstellt, ist mit einem + angedeutet, daß das zweite Arbeitselement 16 die eine Elektrode und mit einem - angedeutet, daß das erste Arbeitselement 14 die Gegenelektrode bildet, d.h. das Arbeitselement 16 und das Arbeitselement 14 befinden sich auf unterschiedlichen Potentialen.

Da das erste Arbeitselement 14 und das zweite Arbeitselement 16 voneinander beabstandet sind, sind zwischen dem ersten Arbeitselement 14 und dem zweiten Arbeitselement 16 keine Isolierungsmaßnahmen notwendig, wodurch sich die Anordnung mit zwei Seite an Seite voneinander beabstandet angeordneten Arbeitselementen 14 und 16 für schmal bauende bipolare Instrumente besonders bewährt hat.

Beim Beaufschlagen des ersten Arbeitselements 14 und des zweiten Arbeitselements 16 mit Strom kann somit zwischen den Innenseiten 40 und 42 bzw. 44 und 46 gefaßtes Gewebe unter der Wirkung des Hochfrequenzstroms koaguliert werden, wobei die Koagulationswirkung auf einen Zwischenraum 50 zwischen dem ersten Arbeitselement 14 und dem zweiten Arbeitselement 16 beschränkt ist, so daß außerhalb dieses Bereichs befindliches Gewebe durch den Hochfrequenzstrom nicht beeinträchtigt wird. In Bereichen, die außerhalb des ersten Arbeitselements 14 und des zweiten Arbeitselements liegen, wie mit 52 angedeutet, fließt kein Strom, und entsprechend tritt dort auch keine Koagulation von Gewebe auf.

Das Instrument 10 weist nun eine dritte Funktion auf, nämlich können mit dem Instrument 10 mit dem ersten Arbeit selement 14 und dem zweiten Arbeitselement 16 gefaßtes Gewebe und auch Gefäße, die in dem Gewebe vorhanden sind, elektrisch unter der Wirkung des Hochfrequenzstroms geschnitten bzw. durchtrennt werden.

Dazu weist zumindest eines der Arbeitselemente 14 oder 16, im Ausführungsbeispiel gemäß Fig. 2 sowohl das erste Arbeitselement 14 als auch das zweite Arbeitselement 16, jeweils zumindest einen, im Ausführungsbeispiel gemäß Fig. 2 jeweils zwei Vorsprünge auf. Dabei weisen das Maulteil 18 des Arbeitselements 14 und das Maulteil 22 des Arbeitselements 16 jeweils einen gegenüberliegenden Vorsprung und das Maulteil 20 des Arbeitselements 14 und das Maulteil 24 des Arbeitselements 16 einen gegenüberliegenden Vorsprung auf, wobei der Vorsprung des Maulteils 18 und der Vorsprung des Maulteils 20 desselben Arbeitselements 14 in Arbeitselementlängsrichtig auf gleicher Höhe und ebenso der Vorsprung der Maulteile 22 und 24 des zweiten Arbeitselements 16 in Arbeitselementlängsrichtung auf gleicher Höhe liegen.

Ein erster Vorsprung 54 ist an dem ersten Maulteil 18, und ein zweiter Vorsprung 56 ist an dem zweiten Maulteil 20 des ersten Arbeitselements 14, und ein erster Vorsprung 58 ist an dem ersten Maulteil 22 und ein zweiter Vorsprung 60 an dem zweiten Maulteil 24 des zweiten Arbeitselements 16 angeordnet.

Die Vorsprünge 54 und 56 des ersten Arbeitselements 14 sind zu dem zweiten Arbeitselement 16 hin gerichtet, und in umgekehrter Weise sind die Vorsprünge 58 und 60 zu dem ersten Arbeitselement 14 hin gerichtet.

Jedes freie Ende der Vorsprünge 54 bis 60 ist nun derart ausgebildet, daß an diesen freien Enden eine Konzentration der Stromdichte des Hochfrequenzstroms auftritt. Dies wird durch die Form der Vorsprünge 54 bis 60, genauer gesagt der freien Enden dieser Vorsprünge 54 bis 60, erreicht, indem die freien Enden der Vorsprünge 54 bis 60 eine Spitze aufweisen, so daß die elektrische Feldstärke an den Spitzen im Vergleich zu den übrigen Bereichen des ersten Arbeitselements 14 und des zweiten Arbeitselements 16 ein Maximum erreicht.

Die Vorsprünge 54 bis 60 sind dabei so positioniert, daß die Vorsprünge 54 und 58 und die Vorsprünge 56 und 60 sich unmittelbar mit einem minimalen Abstand gegenüberstehen.

In Fig. 5 sind mit gestrichelten Linien 62 und 64 die Stromlinien mit maximaler Konzentration der Stromdichte angedeutet. Entlang dieser Stromlinien können mit dem ersten Arbeitselement 14 und dem zweiten Arbeitselement 16 Gewebe und darin enthaltene Gefäße unter Wirkung des Hochfrequenzstroms aufgrund der maximalen Konzentration der Stromdichte geschnitten werden. Zum Schneiden eines Gefäßes wird das Instrument 10 so gehalten, daß die Stromlinien quer zur Längsachse des Gefäßes verlaufen.

Bei dem in Fig. 5 gezeigten Ausführungsbeispiel sind die Vorsprünge 54 bis 60 quer zur Maullängsrichtung des ersten Arbeitselements 14 und des zweiten Arbeitselements 16 gerichtet, wobei das Instrument 10 zum Schneiden eines Gefäßes so gehalten wird, daß sich das Gefäß zwischen den Arbeitselementen 14 und 16 befindet und sich parallel zu diesen erstreckt.

Des weiteren sind alle vier Vorsprünge 54 bis 60 in Maullängsrichtung auf gleicher Höhe angeordnet. Die Positionierung der Vorsprünge 54 und 56 bzw. 58 und 60 ermöglicht ein Schneiden des Gewebes von beiden Seiten her, und zwar entlang der definierten Schnittlinien 62 und 64.

Es versteht sich, daß die Vorsprünge 54 bis 60 metallisch ausgebildet sind und mit dem jeweiligen Maulteil 18 bis 24 elektrisch leitend verbunden sind. Die Vorsprünge 54 bis 60 können bei der Herstellung der Maulteile 18 bis 24 in einem materialabtragenden Formgebungsverfahren ausgebildet werden, wenn die Maulteile 18 bis 24 aus einem Vollmaterial herausgearbeitet werden.

Mit dem Instrument 10 und den Arbeitselementen 14 und 16 gemäß Fig. 5a und 5b wird Gewebe wie folgt präpariert.

Bei geöffneten Maulteilen 18 und 20 bzw. 22 und 24 wird zunächst das zu präparierende Gewebe zwischen die Maulteile 18 und 20 bzw. 22 und 24 gebracht, wonach die Maulteile 18 und 20 bzw. 22 und 24 geschlossen werden.

Unter der Wirkung des bipolaren Hochfrequenzstroms, der bei angeschlossener und eingeschalteter Hochfrequenzspannungsquelle zwischen dem ersten Arbeitselement 14 und dem zweiten Arbeitselement 16 fließt, wird das mit den Maulteilen 18 und 20 bzw. 22 und 24 gefaßte Gewebe koaguliert. Soll ein Gefäß in dem Gewebe koaguliert werden, wird das Gewebe so gefaßt, daß sich das Gefäß quer zu den Arbeitselementen 14 und 16 erstreckt.

Um das Gewebe anschließend zu schneiden oder ein darin befindliches Gefäß zu durchtrennen, werden die Maulteile 18 und 20 bzw. 22 und 24 wieder geöffnet, und das Instrument 10 wird insgesamt etwas zurückgezogen, um das gefaßte Gewebe loszulassen und anschließend werden die Maulteile 18 und 20 bzw. 22 und 24 geschlossen.

Hiernach wird das Instrument 10 um etwa 90° um seine Längsachse gedreht, wonach dann mit geschlossenen Maulteilen 18 und 20 bzw. 22 und 24 durch Vorschieben des Instruments 10 mittels der die sich zwischen den Vorsprüngen 54 und 58 bzw. 56 und 60 ausbildenden Schnittlinien 62 und 64 geschnitten werden kann.

Aus der vorstehenden Beschreibung ergibt sich, daß das Instrument 10 aufgrund der Ausgestaltung des ersten Arbeitselements 14 und des zweiten Arbeitselements 16 die drei Funktionen Fassen, Koagulieren und Schneiden von Gewebe in sich vereint.

Die Vorsprünge 54 und 58 bzw. 56 und 60 sind jeweils mit ihren freien Enden minimal, etwa 1 mm, voneinander beabstandet.

Dadurch, daß sowohl an dem ersten Maulteil 18 als auch an dem zweiten Maulteil 20 jeweils ein Vorsprung 54 bzw. 56 vorgesehen ist, ebenso wie an den Maulteilen 22 und 24 des zweiten Arbeitselements 16, wird zwischen dem ersten Arbeitselement 14 und dem zweiten Arbeitselement 16 eine "elektrische Schneide" ausgebildet, die eine Höhe aufweist, die etwa dem Abstand zwischen den Vorsprüngen 54 und 56 einerseits und den Vorsprüngen 58 und 60 andererseits entspricht.

In Fig. 3 ist ein gegenüber Fig. 2 abgewandeltes Ausführungsbeispiel für ein erstes Arbeitselement 66 und ein zweites Arbeitselement 68 dargestellt, die jeweils wie die Arbeitselemente 14 und 16 als Maul ausgebildet sind, wobei sowohl das erste Arbeitselement 66 als auch das zweite Arbeitselement 68 jeweils vier Vorsprünge 70, d.h. insgesamt acht Vorsprünge 70, aufweisen. Bei dieser Ausgestaltung ergeben sich in Arbeitselementlängsrichtung gesehen zwei voneinander beabstandete Schnittlinien 72 und 74.

Ein weiterer Unterschied zwischen dem ersten Arbeitselement 66 und dem zweiten Arbeitselement 68 gemäß Fig. 3 und dem ersten Arbeitselement 14 und dem zweiten Arbeitselement 16 gemäß Fig. 2 besteht darin, daß das erste Arbeitselement 66 und das zweite Arbeitselement 68 nur jeweils ein bewegliches Maulteil und jeweils ein unbewegliches Maulteil aufweisen, wodurch die jeweiligen Maulteile jedoch wiederum relativ zueinander beweglich sind, wie im Ausführungsbeispiel gemäß Figuren 2 und 1.

In Figuren 4 und 6 ist ein noch weiteres Ausführungsbeispiel für ein erstes Arbeitselement 76 und ein zweites Arbeitselement 78 dargestellt.

Bei diesem Ausführungsbeispiel weisen das erste Arbeitselement 76 und das zweite Arbeitselement 78 jeweils nur einen Vorsprung 80 und 82 auf, wobei der Vorsprung 80 und der Vorsprung 82 jeweils an einem Maulteil des ersten Arbeitselements 76 und des zweiten Arbeitselements 78 angeordnet sind, die sich diametral gegenüberliegen. Auf diese Weise wird eine Schnittlinie 84 erzeugt, die bezüglich der Hauptebene des ersten Arbeitselements 76 und des zweiten Arbeitselements 78 schräg verläuft, d.h. bezüglich der Horizontalen geneigt.

Ein weiteres Ausführungsbeispiel für ein erstes Arbeitselement 86 und ein zweites Arbeitselement 88 ist in Fig. 7 dargestellt, das sich von den vorherigen Ausführungsbeispielen dadurch unterscheidet, daß das erste Arbeitselement 86 das zweite Arbeitselement 88 in Arbeitselementlängsrichtung überragt. Das erste Arbeitselement 86 und das zweite Arbeitselement 88 sind weiterhin Seite an Seite voneinander beabstandet angeordnet, jedoch ist das erste Maul 86 L-förmig ausgebildet, wobei ein quer zur Arbeitselementlängsrichtung stehender Abschnitt 90 vor dem äußersten distalen Ende des Arbeitselements 88 angeordnet ist.

Das erste Arbeitselement 86 und das zweite Arbeitselement 88 weisen jeweils zwei Vorsprünge 92 und 94 bzw. 96 und 98 auf, die im Unterschied zu den vorherigen Ausführungsbeispielen in Arbeitselementlängsrichtung gerichtet sind, so daß Schnittlinien 100 und 102 ebenfalls in Arbeitselementlängsrichtung gerichtet sind. Mit dieser Anordnung der Vorsprünge 92 bis 98 läßt sich mit dem ersten Arbeitselement 86 und dem zweiten Arbeitselement 88 ein Gefäß, das sich quer zur Arbeitselementlängsrichtung erstreckt, durch Drehen des Instruments um seine Längsachse oder durch Bewegen des Arbeitselements in Richtung quer zur Längsrichtung durchtrennen.

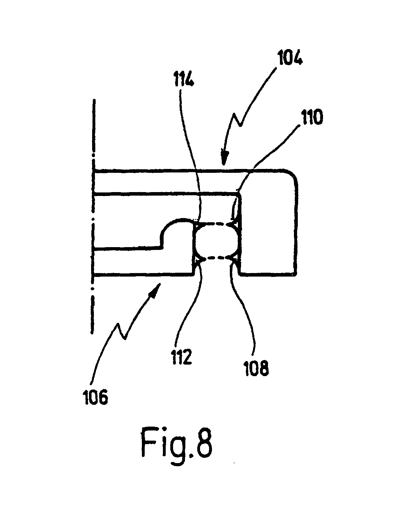

Fig. 8 zeigt ein gegenüber der Fig. 7 leicht abgewandeltes Ausführungsbeispiel, bei dem jedes Maulteil des ersten Arbeitselements 104 und jedes Maulteil des zweiten Arbeitselement 106 zwei nebeneinander angeordnete Vorsprünge 108 und 110 bzw. 112 und 114 aufweist.

Alle die vorgenannten Ausführungsbeispiele können bei dem Instrument 10 in Fig. 1 Verwendung finden. Dazu ist es bei dem Instrument 10 vorgesehen, das erste Arbeitselement 14 und das zweite Arbeitselement 16 abnehmbar auszugestalten, um so anstelle des ersten Arbeitselements 14 und des zweiten Arbeitselements 16 die in Figuren 3 bis 8 dargestellten Mäuler 66, 68; 76, 78; 86, 88 oder 104 und 106 zu montieren.

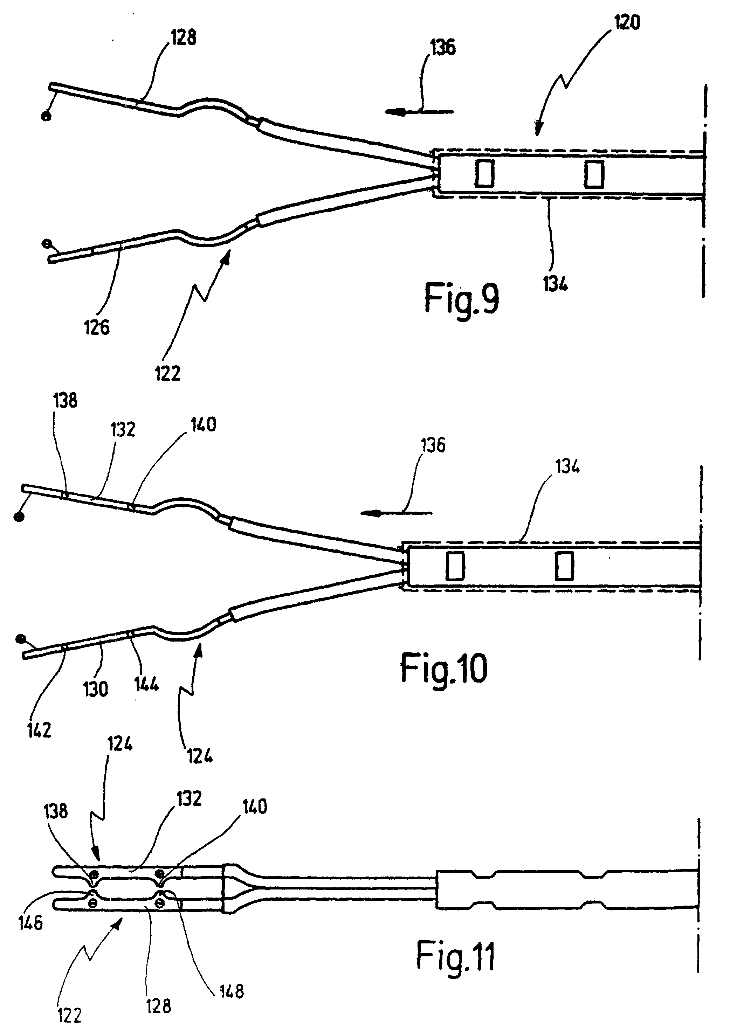

In Fig. 9 bis 11 ist ein weiteres. Ausführungsbeispiel für ein bipolares Instrument zum Schneiden von Gewebe dargestellt, wobei das Instrument 120 wie das Instrument 10 ebenfalls die Funktionen Fassen, Koagulieren und Schneiden in sich vereint.

Das Instrument 120 weist ein erstes Arbeitselement 122 sowie ein zweites Arbeitselement 124 auf, die jeweils als Maul ausgebildet sind. In Fig. 9 ist die Außenseite des ersten Arbeitselements 122 (linkes Arbeitselement) zu sehen, während in Fig. 10 die Innenseite des zweiten Arbeitselements 124 (rechtes Arbeitselement) zu sehen ist.

Das erste Arbeitselement 122 weist ein erstes Maulteil 126 und ein zweites Maulteil 128 auf. Ebenso weist das zweite Arbeitselement 124 ein erstes Maulteil 130 und ein zweites Maulteil 132 auf.

Das Maulteil 126 und das Maulteil 128 des ersten Arbeitselements 122 sind relativ zueinander beweglich, d.h. können aus der in Fig. 9 dargestellten Offenlage in eine Schließlage aufeinander zu bewegt werden. Entsprechendes gilt für die Maulteile 130 und 132 des zweiten Arbeitselements 124.

Der Unterschied zwischen dem ersten Arbeitselement 122 bzw. zweiten Arbeitselement 124 und dem ersten Arbeitselement 14 bzw. dem zweiten Arbeitselement 16 des Instruments 10 besteht darin, daß die Maulteile 18 und 20 bzw. 22 und 24 des Instruments 10 über ein Gelenk miteinander verbunden sind, während die Maulteile 126 und 128 bzw. 130 und 132 als sich federnd aufspreizende Elemente ausgebildet sind, die durch eine Relativbewegung zu einem in Fig. 9 und 10 mit unterbrochenen Linien angedeuteten Schieberohr 134 zusammengedrückt werden können. Bei einer Bewegung des Schieberohrs 134 relativ zu den Maulteilen 126 und 128 bzw. 130 und 132 in Richtung eines Pfeils 136 werden die Maulteile 126 bis 132 geschlossen, und öffnen sich selbsttätig wieder durch eine umgekehrte Bewegung des Schieberohrs 134 relativ zu den Maulteilen 126 bis 132. Dieser Öffnungs- und Schließmechanismus ist bereits bspw. aus dem deutschen Gebrauchsmuster 298 23 913 bekannt, auf das hinsichtlich weiterer Einzelheiten hinsichtlich des Bewegungemechanismus verwiesen wird.

Ähnlich zur Ausgestaltung in Fig. 3 weisen die Maulteile 126 bis 132 auf ihren einander gegenüberliegenden Seiten jeweils zwei Vorsprünge 138 und 140 (Maulteil 132), 142 und 144 (Maulteil 130), 146 und 148 (Maulteil 128) sowie zwei entsprechende, in den Figuren nicht sichtbare Vorsprünge am Maulteil 126 auf. Die Arbeitselemente 122 und 124 bilden eine Elektrode und eine Gegenelektrode, wie mit ⊕ und ⊖ angedeutet ist.

Hinsichtlich der Schneidfunktion des Instruments 120 entspricht die Ausgestaltung der Arbeitselemente 122 und 124 den Arbeitselementen 66 und 68 in Fig. 3, so daß auf die dortigen Ausführungen verwiesen werden kann.

Im Rahmen der Erfindung ist es, wie die vorhergehenden Ausführungsbeispiele zeigen, möglich, durch entsprechende Wahl der Anzahl und Positionierung von Vorsprüngen der Anwendung entsprechende Schnittlinien zum elektrischen Schneiden von Gewebe und Gefäßen zu erzeugen.

Die in den gezeigten Ausführungsbeispielen dargestellte Form der Vorsprünge, die als warzenartig bezeichnet werden kann, und die Anzahl der Vorsprünge ist nur beispielhaft zu verstehen, und es können beliebige andere Formen und Anzahlen von Vorsprüngen gewählt werden, wenn diese geeignet sind, an ihrem freien Ende eine Konzentration der Stromdichte des Hochfrequenzstroms zu bewirken, um elektrisch schneiden zu können.

Die freien Enden der Vorsprünge können anstelle von scharfen Spitzen auch abgerundet sein, oder es können Vorsprünge mit klingenartigen länglichen Kanten vorgesehen sein.

## Patentansprüche

1. Medizinisches bipolares Instrument zum Schneiden von Gewebe, mit einem ersten Arbeitselement (14; 66; 76; 86; 104: 122) und zumindest einem zweiten Arbeitselement (16; 68; 78; 88; 106; 124), die einander benachbart an einem distalen Ende des Instruments (10; 120) angeordnet sind, wobei das erste Arbeitselement (14; 66; 76; 86; 104; 122) und das zweite Arbeitselement (16; 68; 78; 88; 106; 124) als mit Hochfrequenzstrom beaufschlagbare Elektrode und Gegenelektrode ausgebildet sind, wobei das zweite Arbeitselement (16; 68; 78; 88; 106; 124) die eine Elektrode und das erste Arbeitselement (14; 66; 76; 86; 104; 122) die Gegenelektrode bildet, wobei ferner das erste Arbeitselement (14; 66; 76; 86; 104; 122) und das zweite Arbeitselement (16; 68; 78; 88; 106; 124) jeweils als Maul ausgebildet sind, die Seite an Seite voneinander beabstandet angeordnet sind, wobei das erste Maul und das zweite Maul jeweils zwei relativ zueinander bewegliche Maulteile (18, 20, 22, 24; 126, 128, 130, 132) aufweisen, zwischen denen Gewebe gefaßt und koaguliert werden kann, **dadurch gekennzeichnet, daß** ein Manlteil (18; 20; 22; 24; 126; 128; 130; 132) eines der beiden Arbeitselemente (14;66;76;86;104;122; 16; 68; 78; 88; 106; 124) zumindest einen Vorsprung (54-60;) 70; 80; 82; 92-98; 108-114; 138-148) aufweist, der zu dem anderen Arbeitselement (14, 16; 66, 68; 76, 78; 86, 88; 104, 106; 122, 124) hin gerichtet und dessen freies Ende derart ausgebildet ist, daß an diesem eine Konzentration der Stromdichte auftritt.

2. Instrument nach Anspruch 1, **dadurch gekennzeichnet, daß** sowohl das erste Arbeitselement (14; 66; 76; 86; 104; 122) als auch das zweite Arbeitselement (16; 68; 78; 88; 106; 124) jeweils zumindest einen Vorsprung (54-60; 70; 80; 82; 92-98; 108-114; 138-148) aufweisen.

3. Instrument nach Anspruch 2, **dadurch gekennzeichnet, daß** der zumindest eine Vorsprung (54-60; 70; 80; 82; 92-98; 108-114; 138-148) des ersten Arbeitselements (14; 66; 76; 86; 104; 122) dem zumindest einen Vorsprung des zweiten Arbeitselements (16; 68; 78; 88; 106; 124) gegenübersteht.

4. Instrument nach einem der Ansprüche 1 bis 3, **dadurch gekennzeichnet, daß** der zumindest eine Vorsprung (54-60; 70; 80; 82; 138-148) schräg zur Arbeitselementlängsrichtung gerichtet ist.

5. Instrument nach einem der Ansprüche 1 bis 4, **dadurch gekennzeichnet, daß** das erste Arbeitselement (86; 104) das zweite Arbeitselement (88; 106) in Arbeitselementlängsrichtung überragt, und daß der zumindest eine Vorsprung (92-98; 108-114) in Arbeitselementlängsrichtung gerichtet ist.

6. Instrument nach einem der Ansprüche 1 bis 5, **dadurch gekennzeichnet, daß** das freie Ende des zumindest einen Vorsprungs (54-60; 70; 80; 82; 92-98; 108-114; 138-148) als Spitze ausgebildet ist.

7. Instrument nach einem der Ansprüche 1 bis 6, **dadurch gekennzeichnet, daß** das freie Ende des zumindest einen Vorsprungs abgerundet ist.

8. Instrument nach einem der Ansprüche 2 bis 7, **dadurch gekennzeichnet, daß** das freie Ende des zumindest einen Vorsprungs (54-60; 70; 80; 82; 92-98; 108-114; 138-148) des ersten Arbeitselements (14; 66; 76; 86; 104; 122) und das freie Ende des gegenüberliegenden Vorsprungs (54-60; 70; 80; 82; 92-98; 108-114; 138-148) des zweiten Arbeitselements (16; 68; 78; 88; 106; 124) einen Abstand voneinander aufweisen, der kleiner ist als der Abstand der Arbeitselemente (14, 16; 66, 68; 76, 78; 104, 106; 122, 124; ) in deren übrigem Bereich voneinander.

9. Instrument nach einem der Ansprüche 1 bis 8, **dadurch gekennzeichnet, daß** das freie Ende des zumindest einen Vorsprungs als längliche Kante ausgebildet ist.

10. Instrument nach Anspruch 9, **dadurch gekennzeichnet, daß** der zumindest eine Vorsprung keilartig schräg zur Arbeitselementlängsrichtung verläuft.

11. Instrument nach Anspruch 10, **dadurch gekennzeichnet, daß** sowohl an dem ersten Arbeitselement als auch an dem zweiten Arbeitselement zumindest ein als längliche Kante ausgebildeter Vorsprung vorgesehen ist, wobei die Kanten jeweils schräg zur Arbeitselementlängsrichtung verlaufend ausgebildet sind, wobei die sich gegenüberliegenden Kanten von distal nach proximal gesehen gegensinnig schräg zueinander verlaufen.

12. Instrument nach einem der Ansprüche 1 bis 11, **dadurch gekennzeichnet, daß** der zumindest eine Vorsprung (54, 58; 70; 92, 96; 112, 114; 138, 140) an dem ersten Maulteil (18, 22; 130, 132) des einen Arbeitselements (14, 16; 66, 68; 76, 78; 86, 88; 104, 106; 122, 124) angeordnet ist, und daß zumindest ein zweiter Vorsprung (56, 60; 70; 94, 98; 112, 114; 142, 144) an dem zweiten Maulteil (20, 24; 130, 132) desselben Arbeitselements (14, 16; 66, 68; 76, 78; 86, 88; 104, 106; 122, 124) angeordnet ist.

13. Instrument nach einem der Ansprüche 1 bis 12, **dadurch gekennzeichnet, daß** der zumindest eine Vorsprung (80) des einen Arbeitselements (76) an dem ersten Maulteil dieses Arbeitselements (76) und der zumindest eine Vorsprung (82) des zweiten Arbeitselements (78) an demjenigen Maulteil des anderen Arbeitselements (78) angeordnet ist, das dem ersten Maulteil des ersten Arbeitselements (76) diametral gegenüberliegt.

14. Instrument nach einem der Ansprüche 12 bis 13, **dadurch gekennzeichnet, daß** die zumindest beiden Vorsprünge (54-60; 70; 80; 82; 92-98; 138-148) in Arbeitselementlängsrichtung auf der gleichen Höhe angeordnet sind.

15. Instrument nach einem der Ansprüche 1 bis 14, **dadurch gekennzeichnet, daß** das erste Arbeitselement (14; 66; 76; 86; 104; 122) und das zweite Arbeitselement (16; 68; 78; 88; 106; 124) auswechselbar sind.

## Claims

1. A medical bipolar instrument for cutting tissue, comprising a first working element (14; 66; 76; 86; 104; 122) and at least one second working element (16; 68; 78; 88; 106; 124), which are arranged adjacent to one another at a distal end of the instrument (10; 120), wherein the first working element (14; 66; 76; 86; 104; 122) and the second working element (16; 68; 78; 88; 106; 124) are configured as an electrode and a counter electrode suppliable with high frequency current, the second working element (16; 68; 78; 88; 106; 124) forming one electrode and the first working element (14; 76; 76; 86; 104; 122) forming the counter electrode, the first working element (14; 66; 76; 86; 104; 122) and the second working element (16; 68; 78; 88; 106; 124) further being configured as jaws which are arranged side by side at a spacing from one another, the first jaw and the second jaw each having two jaw parts (18, 20, 22, 24; 126, 128, 130, 132), between which tissue can be grasped and coagulated, **characterized in that** at least one jaw part (18; 20; 22; 24; 126; 128; 130; 132) of one of both working elements (14; 66; 76; 86; 104; 122; 16; 68; 78; 88; 106; 124) comprises a projection (54-60; 70; 80; 82; 92-98; 108-144; 138-148), which is directed toward the other working element (14, 16; 66, 68; 76, 78; 86; 88; 104, 106; 122, 124) and its free end is configured such that a concentration of the current density occurs at same.

2. The instrument of claim 1, **characterized in that** both the first working element (14; 66; 76; 86; 104; 122) and the second working element (16; 68; 78; 88; 106; 124) each comprise at least one projection (54-60; 70; 80; 82; 92-98; 108-114; 138-148).

3. The instrument of claim 2, **characterized in that** the at least one projection (54-60; 70; 80; 82; 92-98; 108-114; 138-148) of the first working element (14; 66; 76; 86, 104; 122) opposes the at least one projection of the second working element (16; 68; 78; 88; 106; 124).

4. The instrument of anyone of claims 1 through 3, **characterized in that** the at least one projection (54-60; 70; 80; 82; 138-148) is directed to be inclined with respect to the working element longitudinal direction.

5. The instrument of anyone of claims 1 through 4, **characterized in that** the first working element (86; 104) extends beyond the second working element (88; 106) in the working element longitudinal direction and the at least one projection (92-98) is directed in working element longitudinal direction.

6. The instrument of anyone of claims 1 through 5, **characterized in that** the free end of the at least one projection (54-60; 70; 80; 82; 92-98; 108-114; 138-148) is configured as a point.

7. The instrument of anyone of claims 1 through 6, **characterized in that** the free end of the at least one projection is rounded.

8. The instrument of anyone of claims 2 through 7, **characterized in that** the free end of the at least one projection (54-60; 70; 80; 82; 92-98; 108-114; 138-148) of the first working element (14; 66; 76; 86; 104; 122) and the free end of the opposite projection (54-60; 70; 82; 92-98; 108-114; 138-148) of the second working element (16; 68; 78; 88; 106; 124) have a spacing from one another, which is smaller than the spacing of the working elements (14, 16; 66, 68; 76, 78; 104, 106; 122, 124) from one another in their remaining region.

9. The instrument of anyone of claims 1 through 8, **characterized in that** the free end of the at least one projection is configured as an elongated edge.

10. The instrument of claim 9, **characterized in that** the at least one projection runs in wedge-like manner at an inclination with respect to the working element longitudinal direction.

11. The instrument of claim 10, **characterized in that** both the first working element and the second working element have at least one projection formed as an elongated edge, wherein each of the edges are formed to run at an inclination with respect to the working element longitudinal direction, and wherein the opposing edges run counter directionally inclined to one another when seen from distally to proximally.

12. The instrument of anyone of claims 1 through 11, **characterized in that** the at least one projection (54, 58; 70; 92, 96; 112, 114; 138, 140) is arranged on the first jaw part (18, 22; 130, 132) of one working element (14, 16; 66, 68; 76, 78; 86, 88; 104, 106; 122, 124) and the at least one second projection (56, 60; 70; 94, 98; 112, 114; 142, 144) is arranged on the second jaw part (20, 24; 130, 132) of the same working element (14, 16; 66, 68; 76, 78; 86, 88; 104, 106; 122, 124).

13. The instrument of anyone of claims 1 through 12, **characterized in that** the at least one projection (80) of one working element (76) is arranged on the first jaw part of this working element (76) and the at least one projection (82) of the second working element (78) is arranged on that jaw part of the other working element (78), which lies diametrically opposite to the first jaw part of the first working element (76).

14. The instrument of anyone of claims 12 through 13, **characterized in that** the at least two projections (54-60; 70; 80; 82; 92-98; 138-148) are arranged at the same position in working element longitudinal direction.

15. The instrument of anyone of claims 1 through 14, **characterized in that** the first working element (14; 66; 76; 86; 104; 122) and the second working element (16; 68; 78; 88; 106; 124) are exchangeable.

## Revendications

1. Instrument médical bipolaire pour découper des tissus, avec un premier élément de travail (14 ; 66 ; 76 ; 86 ; 104 ; 122) et au moins un deuxième élément de travail (16 ; 68 ; 78 ; 88 ; 106 ; 124), qui sont placés l'un près de l'autre sur une extrémité distale de l'instrument (10 ; 120), le premier élément de travail (14 ; 66 ; 76 ; 86 ; 104 ; 122) et le deuxième élément de travail (16 ; 68 ; 78 ; 88 ; 106 ; 124) étant conformés en électrode et contre-électrode pouvant être exposées à un courant à haute fréquence, le deuxième élément de travail (16 ; 68 ; 78 ; 88 ; 106 ; 124) formant la première électrode et le premier élément de travail (14 ; 66 ; 76 ; 86 ; 104 ; 122) la contre-électrode, le premier élément de travail (14 ; 66 ; 76 ; 86 ; 104 ; 122) et le deuxième élément de travail (16 ; 68 ; 78 ; 88 ; 106 ; 124) étant chacun conformés de plus en mâchoires qui sont placées côté à côté à distance l'une de l'autre, la première mâchoire et la deuxième mâchoire présentant chacune deux parties de mâchoire (18, 20, 22, 24 ; 126, 128, 130, 132) mobiles l'une par rapport à l'autre, entre lesquelles un tissu peut être saisi et coagulé, **caractérisé en ce qu'**au moins une partie de mâchoire (18 ; 20 ; 22 ; 24 ; 126 ; 128 ; 130 ; 132) de l'un des deux éléments de travail (14 ; 66 ; 76 ; 86 ; 104 ; 122 ; 16 ; 68 ; 78 ; 88 ; 106 ; 124) présente une saillie (54 ; 60 ; 70 ; 80 ; 82 ; 92-98 ; 108-114 ; 138-148), qui est tournée vers l'autre élément de travail et dont l'extrémité libre est conformée de telle manière qu'une concentration de la densité de courant se produit sur elle.

2. Instrument selon la revendication 1, **caractérisé en ce que** le premier élément de travail (14 ; 66 ; 76 ; 86 ; 104 ; 122) et le deuxième élément de travail (16 ; 68 ; 78 ; 88 ; 106 ; 124) présentent chacun au moins une saillie (54-60 ; 70 ; 80 ; 82 ; 92-98 ; 108-114 ; 138-148).

3. Instrument selon la revendication 2, **caractérisé en ce que** la saillie (54-60 ; 70 ; 80 ; 82 ; 92-98 ; 108-114 ; 138-148) au nombre d'au moins une du premier élément de travail (14 ; 66 ; 76 ; 86 ; 104 ; 122) fait face à la saillie au nombre d'au moins une du deuxième élément de travail (16 ; 68 ; 78 ; 88 ; 106 ; 124).

4. Instrument selon l'une des revendications 1 à 3, **caractérisé en ce que** la saillie (54-60 ; 70 ; 80 ; 82 ; 138-148) au nombre d'au moins une est dirigée à l'oblique par rapport à la direction longitudinale de l'élément de travail.

5. Instrument selon l'une des revendications 1 à 4, **caractérisé en ce que** le premier élément de travail (86 ; 104) dépasse du deuxième élément de travail (88 ; 106) dans la direction longitudinale de l'élément de travail, et **en ce que** la saillie (92-98 ; 108-114) au nombre d'au moins une est dirigée dans la direction longitudinale de l'élément de travail.

6. Instrument selon l'une des revendications 1 à 5, **caractérisé en ce que** l'extrémité libre de la saillie (54-60 ; 70 ; 80 ; 82 ; 92-98 ; 108-114 ; 138-148) au nombre d'au moins une est conformée en pointe.

7. Instrument selon l'une des revendications 1 à 6, **caractérisé en ce que** l'extrémité libre de la saillie au nombre d'au moins une est arrondie.

8. Instrument selon l'une des revendications 2 à 7, **caractérisé en ce que** l'extrémité libre de la saillie (54-60 ; 70 ; 80 ; 82 ; 92-98 ; 108-114 ; 138-148) au nombre d'au moins une du premier élément de travail (14 ; 66 ; 76 ; 86 ; 104 ; 122) et l'extrémité libre de la saillie opposée (54-60 ; 70 ; 80 ; 82 ; 92-98 ; 108-114 ; 138-148) du deuxième élément de travail (16 ; 68 ; 78 ; 88 ; 106 ; 124) présentent entre elles une distance qui est inférieure à la distance entre les éléments de travail (14, 16 ; 66, 68 ; 76, 78 ; 104, 106 ; 122, 124) dans la partie restante.

9. Instrument selon l'une des revendications 1 à 8, **caractérisé en ce que** l'extrémité libre de la saillie au nombre d'au moins une est conformée en arête oblongue.

10. Instrument selon la revendication 9, **caractérisé en ce que** la saillie au nombre d'au moins une s'étend en forme de coin de manière oblique par rapport à la direction longitudinale de l'élément de travail.

11. Instrument selon la revendication 10, **caractérisé en ce que** tant sur le premier élément de travail que sur le deuxième élément de travail, il est prévu au moins une saillie conformée en arête oblongue, les arêtes s'étendant à chaque fois à l'oblique par rapport à la direction longitudinale de l'élément de travail, les arêtes mutuellement opposées s'étendant à l'oblique l'une par rapport à l'autre et en sens inverse en regardant du côté distal au côté proximal.

12. Instrument selon l'une des revendications 1 à 11, **caractérisé en ce que** la saillie (54, 58 ; 70 ; 92, 96 ; 112, 114 ; 138, 140) au nombre d'au moins une est placée sur la première partie de mâchoire (18, 22 ; 130, 132) d'un élément de travail (14, 16 ; 66, 68 ; 76, 78 ; 86, 88 ; 104, 106 ; 122, 124), et **en ce qu'**au moins une deuxième saillie (56, 60 ; 70 ; 94, 98 ; 112, 114 ; 142, 144) est placée sur la deuxième partie de mâchoire (20, 24 ; 130, 132) du même élément de travail (14, 16 ; 66, 68 ; 76, 78 ; 86, 88 ; 104, 106 ; 122, 124).

13. Instrument selon l'une des revendications 1 à 12, **caractérisé en ce que** la saillie (80) au nombre d'au moins une d'un élément de travail (76) est placée sur la première partie de mâchoire de cet élément de travail (76) et la saillie (82) au nombre d'au moins une du deuxième élément de travail (78) est placée sur la partie de mâchoire de l'autre élément de travail (78) qui est diamétralement opposée à la première partie de mâchoire du premier élément de travail (76).

14. Instrument selon l'une des revendications 12 et 13, **caractérisé en ce que** les saillies (54-60 ; 70 ; 80 ; 82-98 ; 138-148) au nombre d'au moins deux sont placées à la même hauteur dans la direction longitudinale de l'élément de travail.

15. Instrument selon l'une des revendications 1 à 14, **caractérisé en ce que** le premier élément de travail (14 ; 66 ; 76 ; 86 ; 104 ; 122) et le deuxième élément de travail (16 ; 68 ; 78 ; 88 ; 106 ; 124) sont interchangeables.
